Europäisches Patentamt

European Patent Office

Office européen des brevets

(11) Publication number: **0 486 125 A1**

## EUROPEAN PATENT APPLICATION

(21) Application number: **91304636.3**

(22) Date of filing: **22.05.91**

(51) Int. Cl.⁵: **D21H 21/32**, D21C 9/10, D21C 9/00, D21C 5/02, D21C 3/00

The microorganism(s) has (have) been deposited with Fermentation Reseach Institute under number(s) FERM BP-3391.

(30) Priority: **13.11.90 JP 306772/90**

(43) Date of publication of application: **20.05.92 Bulletin 92/21**

(84) Designated Contracting States: **AT BE CH DE DK ES FR GB GR IT LI LU NL SE**

(71) Applicant: **JAPAN PULP & PAPER RESEARCH INSTITUTE, INC. 7-5 Ginza 4-chome Chuo-ku, Tokyo 104(JP)**

(72) Inventor: **Sakamoto, Reiichiro, Japan Pulp & Paper Res.Inst. 13-11 Tokodai 5-chome Tsukuba-shi, Ibaraki 300-26(JP)** Inventor: **Suzuki, Akiko, Japan Pulp & Paper Res.Inst. 13-11 Tokodai 5-chome Tsukuba-shi, Ibaraki 300-26(JP)** Inventor: **Kamaya, Yasushi, Japan Pulp & Paper Res.Inst. 13-11 Tokodai 5-chome Tsukuba-shi, Ibaraki 300-26(JP)**

Inventor: **Konno, Mitsugi, Japan Pulp & Paper Res.Inst. 13-11 Tokodai 5-chome Tsukuba-shi, Ibaraki 300-26(JP)** Inventor: **Suzuki, Masato, Japan Pulp & Paper Res.Inst. 13-11 Tokodai 5-chome Tsukuba-shi, Ibaraki 300-26(JP)** Inventor: **Tamba, Kazuyo, Japan Pulp & Paper Res.Inst. 13-11 Tokodai 5-chome Tsukuba-shi, Ibaraki 300-26(JP)** Inventor: **Iwanaga, Yuzoh, Japan Pulp & Paper Res.Inst. 13-11 Tokodai 5-chome Tsukuba-shi, Ibaraki 300-26(JP)** Inventor: **Kondoh,Masaharu, Japan Pulp & Paper Res.Inst. 13-11 Tokodai 5-chome Tsukuba-shi, Ibaraki 300-26(JP)** Inventor: **Tajiri, Masanao, Japan Pulp & Paper Res.Inst. 13-11 Tokodai 5-chome Tsukuba-shi, Ibaraki 300-26(JP)**

(74) Representative: **Ford, Michael Frederick et al MEWBURN ELLIS 2 Cursitor Street London EC4A 1BO(GB)**

(54) **Lignocellulolytic composition, process of producing the same and use of the same.**

㊼ Bio-kaisen compositions which disintegrate wastepaper or other lignocellulose while substantially not decomposing the cellulose or hemicellulose are disclosed. A bio-kaisen composition according to the present invention has each CM-cellulase and xylanase activity of not more than 0.5IU and a newspaper bio-kaisen rate of not less than 50%.

EP 0 486 125 A1

SPECIFICATION

BACKGROUND OF THE INVENTION

I. Field of the Invention

The present invention relates to a bio-kaisen composition which is used in the pulping process, for example, the processing of secondary fibers from wastepaper or the processing of mechanical, chemical or semichemical pulp from lignocellulose such as wood and nonwood including straw and sugar cane bagasse.

II. Description of the Related Art

In the pulping process, the defibering and beating step for disintegrating the raw materials such as wastepaper or lignocellulose is necessary. These steps is conventionally carried out by mechanically or chemically processing the wastepaper or lignocellulose. However, these processings necessitate a large equipment and the process cost is high.

It has been proposed to empoly a cellulolytic enzyme such as cellulase or xylanase in the secondary fiber processing to remove ink or to improve the drainage of pulp(Japanese Laid Open Patent Application (Kokai) Nos. 9299/84, 59494/88, 145495/88, Tappi, vol. 72(6), p.187 (1989), or in the pulping process to improve the pulp quality, to promote the beating performance of the pulp or to bleachability the pulp. However, if the pulping process is carried out using cellulase or xylanase, the fibers per se made of cellulose and hemicellulose are decomposed with acting enzyme, These enzyme actions take place to lower the pulp strength and to reduse the pulp yield. Therefore, the pulping process utilizing the cellulolytic enzymes are practically very difficult.

SUMMARY OF THE INVENTION

Accordingly, an object of the present invention is to provide the available means for disintegrating the wastepaper or lignocellulose accompanying much lower decomposition of the cellulose or hemicellulose constituting the fibers than occurred in the conventional enzymatic processes, in the defibering or beating step in the pulping process such as the processing of secondary fibers from wastepaper or the processing of mechanical, chemical or semichemical pulp from lignocellulose, and in the fiber refinary process of deinking or bleaching.

The present inventors intensively studied to discover a composition with high bio-kaisen activity while with very low cellulase and xylanase activities, thereby are completing the present invention realized the above purpose.

That is, the present invention provides a bio-kaisen composition having each CM-cellulase and xylanase activity of not more than 0.5 IU and a bio-kaisen activity in terms of the newspaper bio-kaisen rate of not less than 50%.

The present invention also provides a bio-kaisen composition having a CM-cellulase activity of not more than 0.35IU and a bio-kaisen activity in terms of newspaper bio-kaisen rate of not less than 50%.

The present invention further provides a bio-kaisen composition having each CM-cellulase and xylanase activity of not more than 0.14 IU and a bio-kaisen activity in terms of the PPC (plain paper copier) paper bio-kaisen rate of not less than 50%.

The present invention still further provides a bio-kaisen composition having a CM-cellulase activity of not more than 0.05 IU and a bio-kaisen activity in terms of the PPC paper bio-kaisen rate of not less than 50%.

The present invention still further provides a bio-kaisen composition having a bio-kaisen activity in term of the bio-kaisen pulp yield determined by the PPC paper bio-kaisen pulp recovery test of not less than 43%.

The present invention still further provides a bio-kaisen composition having each CM-cellulase and xylanase activity of not more than 400 IU and the bio-kaisen pulp yield of not less than 30%.

The present invention still further provides a bio-kaisen composition having a CM-cellulase activity of not more than 200 IU and the bio-kaisen pulp yield of not less than 30%.

The present invention still further provides a process of producing the bio-kaisen composition, comprising the steps of culturing a microbe belonging to genera Trichoderma and Coriolus, which has an ability to produce the bio-kaisen composition, and of recovering the bio-kaisen composition produced.

The present invention still further provides a process of culturing the microbes in the presence of material contained cellulose or hemicellulose to obtain the bio-kaisen composition.

The present invention still further provides a process of culturing the microbes in the presence of wastepaper, secondary fiber prepared from wastepaper or pulp from lignocellulose to obtain the bio-kaisen composition.

The present invention still further provides a processing method of above various pulp or paper comprising the step of treating those with the bio-kaisen composition.

The present invention still further provides a recovering method of the fibers obtained by processing above various pulp or paper comprising the step of treating those with the bio-kaisen composition.

The present invention still further provides a processing method of above various pulp or paper comprising the step of deinking those with the bio-kaisen composition.

The present invention still further provides a recovering method of the fibers obtained by processing above various pulp or paper comprising the step of deinking those with the bio-kaisen composition.

The present invention still further provides a processing method of above various pulp or paper comprising the step of bleaching those with the bio-kaisen composition.

The present invention still further provides a recovering method of the fibers obtained by processing above various pulp or paper comprising the step of bleaching those with the bio-kaisen composition.

By the present invention, the compositions with high bio-kaisen activity but with low cellulase and xylanase activity, as well as processes of producing the compositions were provided. If the pulping process of wastepaper or pulp is conducted employing the composition of the present invention, the paper or pulp is more thoroughly disintegrated into individual fiber while the obtained fibers are substantially not decomposed or very limitedly hydrolyzed. Thus, by using the bio-kaisen composition, wastepaper or pulp may be more thoroughly disintegrated or beaten almost without lowering the pulp strenghth and reducing the pulp yield. The obtained fibers with high quality may be supplied to the conventional pulping process, that is, the operations such as disintegration, beating, deinking, bleach and refinary of the wastepaper or pulp.

Consequently, the present invention will contributes largely to the field of the pulp and paper industry.

BRIEF DESCRIPTION OF THE DRAWINGS

Fig 1 shows the correlation between the amount of a conventional enzyme added and the newspaper bio-kaisen rate;

Fig. 2 shows the correlation between the amount of a conventional enzyme added and the PPC paper bio-kaisen rate;

Fig. 3 shows the correlation between CM-cellulase activity and bio-kaisen pulp yield; and

Fig. 4 shows the correlation between xylanase activity and bio-kaisen pulp yield.

The term "bio-kaisen" herein means to disintegrate paper or lignocellulosic materials into individual fiber. The bio-kaisen activity is expressed in terms of the newspaper bio-kaisen rate, the PPC paper bio-kaisen rate or the bio-kaisen pulp yield and the CM-cellulase and xylanase activity may be measured by conventional methods, of which definitions are described later in detail.

The bio-kaisen composition according to the first aspect of the present invention has each CM-cellulase and xylanase activity of not more than 0.5 IU, preferably not more than 0.3 IU and a newspaper bio-kaisen rate of not less than 50%.

The bio-kaisen composition according to the second aspect of the present invention has a CM-cellulase activity of not more than 0.35 IU, preferably not more than 0.2 IU and a newspaper bio-kaisen rate of not less than 50%.

The bio-kaisen activity may be determined by a new testing method developed by the present inventor in which the degree of disintegration of newspaper sample disks into fiber is measured in terms of the reduction rate of the weight of the samples. More particularly, the newspaper bio-kaisen activity recited in the first and the second compositions according to the present invention is determined as followed:

Method for Determining Newspaper Bio-kaisen Rate

(i) Bio-kaisen Reaction toward Newspaper

The circular disks prepare by punching a Japanese conventional newspaper (newsprint paper constituted of deinked pulp, mechanical pulp and semibleached kraft pulp) whose one side is painted in black with an offset printing ink with a punch having a diameter of 4.5 mm are used as the substrate of reaction. A six-well multidish (well diameter of 35 mm and well depth of 17.3 mm) is used as a reaction vessel and bio-kaisen compositions appropriately concentrated or diluted to six levels are put into each well in the amount of 2 ml per well. The sample paper disks are immersed in water made 0.2 mg/ml with respect to $NaN_3$ to prevent bacterial growth to sufficiently soak the disks in water. Five disks (about 3.7 mg) are put into the reaction mixture contained the bio-kaisen compositions. Following the wells are sealed with cellophane tape, the multidish is incubated at 37°C for 24 hours on the reciprocal shaker (7 cm excursion diameter, 120 strokes per minute). After incubation, the multidish is taken out of the shaker to terminate the reaction.

(ii) Estimation of Bio-kaisen Activity

After the reaction, 15 ml of water is added to each reaction mixture, and the pulp suspension disintegrated the sample paper disks is filtrated through a glass filter apparatus. This apparatus comprises a 300 ml funnel, 16 mesh stainless screen (Tyler series, openings 1.00 mm, effective diameter of 32 mm) and a one liter bottle. The residue remained on the screen is washed with 50 ml of water. The screen on which the residue remains is separated from the apparatus and dried at 105°C for 2 hours, measured to determine the dry weight of the residue. As a control, the same sample (5 newspaper disks per each well) is incubated with water in the same manner and the dry weight of the residue is determined. The newspaper bio-kaisen rate is estimated as follows:

$$\frac{(A - B)}{A} \times 100 = \text{Bio-kaisen rate (\%)}$$

where

A = weight of oven-dry residue from the control reaction

B = weight of oven-dry residue from the Bio-kaisen reaction

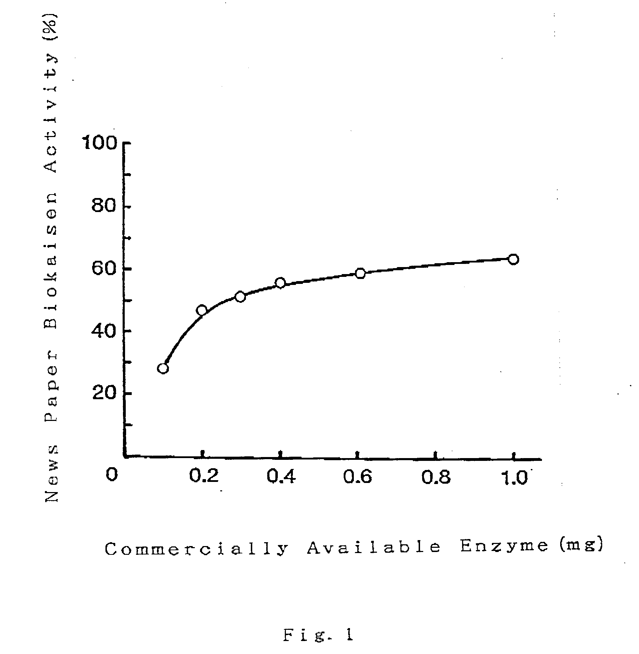

The correlation between the amount of the bio-kaisen composition and the bio-kaisen rate is shown in Fig. 1. The compositions estimated newspaper bio-kaisen rate of not less than 50% is evaluated to have the bio-kaisen activity.

To adjust the amount of the bio-kaisen composition to attain a newspaper bio-kaisen rate of around 50%, the following observation test in which the morphological change of paper disks disintegrated into the fibers is examined is practically effective.

(iii) Observation Test for Estimating Bio-kaisen Rate

After bio-kaisen reaction toward newspaper, the paper disks remaining and fibers disintegrated in the reaction mixture are grossly observed and estimated of the following six ranks for the degree of disintegration of the paper.

Rank 0     No change is observed in the paper disks

Rank 1     Paper disks are not broken but fibers disintegrated is slightly observed in the reaction mixture

Rank 2     Paper disks are slightly broken and a little fiber disintegrated is observed in the reaction mixture

Rank 3     Paper disks are partly broken and some fibers disintegrated is observed in the reaction mixture

Rank 4     Half paper disk are broken and many fibers is peeled from the ink layer in the reaction mixture

Rank 5     The paper disks no longer retain their original shape at all and most fibers is peeled from the ink layer in the reaction mixture

The newspaper bio-kaisen rate of 50% roughly corresponds to the rank 3 or 4 in this observation test. If firstly the observation test are carried out, the amount of the composition may be easily adjusted to attain the newspaper bio-kaisen rate of around 50%.

The bio-kaisen composition according to the third aspect of the present invention has each CM-cellulase and xylanase activity of not more than 0.14 IU, preferably not more than 0.1 IU and a PPC paper bio-kaisen rate of not less than 50%.

The bio-kaisen composition according to the fourth aspect of the present invention has a CM-cellulase activity of not more than 0.05 IU, preferably not more than 0.035 IU and a PPC paper bio-kaisen rate of not less than 50%.

The PPC paper bio-kaisen rate is measure as followed:

Method for Determining PPC Paper Bio-kaisen Rate

The PPC paper bio-kaisen rate is determined in the same manner as the method for newspaper bio-kaisen rate except that the sample is PPC paper disks prepared by punching a conventional PPC paper (rosinsized woodfree paper constituted of bleached kraft pulp from mainly hardwood) whose one side is painted in black by operating a plain paper copier without the face cover of copyingmachine. The weight of five paper disks is about 5.7 mg.

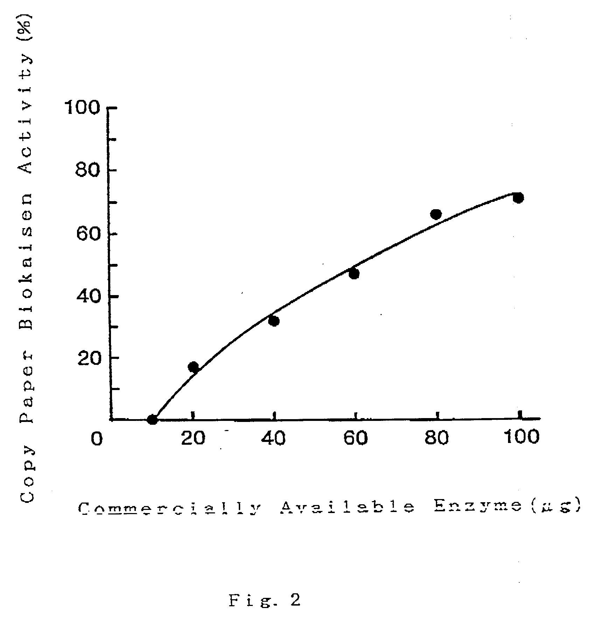

The correlation between the amount of the bio-kaisen composition and the PPC paper bio-kaisen rate is shown in Fig. 2. The compositions estimated PPC paper bio-kaisen rate of not less than 50% is evaluated to have the bio-kaisen activity. The observation test described above is practically effective for determining the PPC paper bio-kaisen rate of around 50% as well.

The bio-kaisen composition according to the fifth aspect of the present invention has a bio-kaisen activity in terms of bio-kaisen pulp yield determined by PPC paper bio-kaisen pulp recovery test of not less 43%, preferably not less than 46%.

The bio-kaisen composition according to the sixth aspect of the present invention has each CM-cellulase and xylanase activity of not more than 400 IU, preferably not more than 300 IU and a bio-kaisen activity in terms of bio-kaisen pulp yield of not less than 30%.

The bio-kaisen composition according to the seventh aspect of the present invention has a CM-cellulase activity of not more than 200 IU, preferably not more than 100 IU and a bio-kaisen activity in terms of bio-kaisen pulp yield of not less than 30%.

The bio-kaisen pulp yield is determined as follows:

Method for Determining PPC Paper Bio-kaisen Pulp Yield

(i) Soaking Reaction on PPC Paper

A conventional PPC paper (rosinsized woodfree paper constituted of bleached kraft pulp from mainly hardwood) copied stock market column of a newspaper by a plain paper copier is used as the substrate for the reaction. The PPC paper is cut into pieces sizing 1 cm x 2 cm. Ten grams of the cut paper pieces are put in a Erlenmeyer flask, 20 ml of 1M acetate buffer, pH 5.0, and 170 ml of appropriately concentrated or diluted bio-kaisen composition are then added. The flask is still incubated at 50°C to soak the sample paper. After one hour, the flask is taken out to terminate the reaction.

(ii) Estimation of Bio-kaisen Pulp Yield

After the reaction, the paper soaked is subjected to the Bauer-McNett classifier in accordance with TAPPI Standard T-233 hm-82, the classification is carried out 10 minutes. Two screens are used and each one of the first and second compartment are 24 mesh (openings 0.710 mm) and 150 mesh (openings 0.105 mm); respectively. Although the most of fibers disintegrated from the sample paper pass through the 24 mesh screen, and fine fraction pass through the 150 mesh screen. Thus, the available fibers remains in the second compartment, the fibers is recovered and the oven-dry weight of those is measured. As a control, the same operation as described above is carried out except that the sample paper are soaked with water. The bio-kaisen pulp yield is estimated as follows:

$$\frac{(C - D)}{10 \text{ g}} \times 100 = \text{Bio-kaisen pulp yield (\%)}$$

where

C = weight of oven-dry fibers remained in the second compartment of the control reaction
D = weight of oven-dry fibers remained in the second compartment of the bio-kaisen reaction

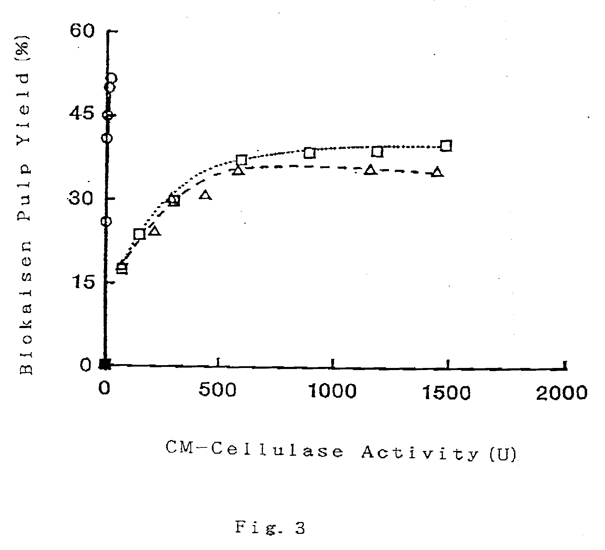

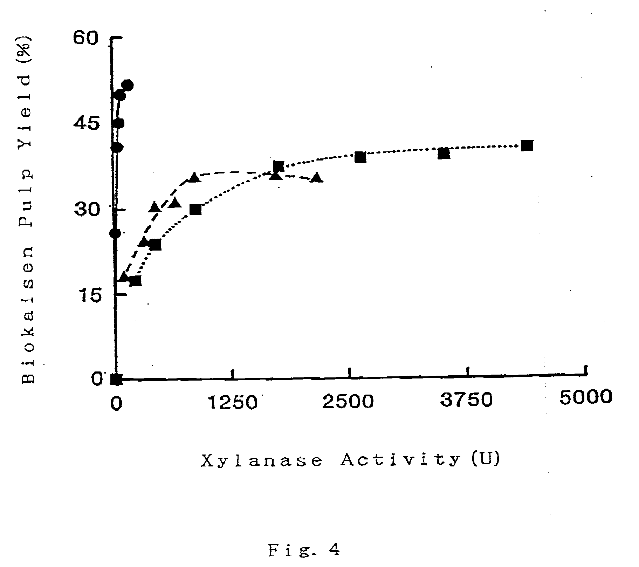

The correlation between the CM-cellulase activity and the bio-kaisen pulp yield is shown in Fig. 3. Similarly, the correlation between the xylanase activity and the bio-kaisen pulp yield is shown in Fig. 4. The conventional cellulolytic enzyme (indicated by triangle) cannot reach the bio-kaisen pulp yield of more than 40%. If the activity of the above conventional enzyme is weak, the bio-kaisen pulp yield is low because the PPC paper is effectively not disintegrated, and even if the activity is strong, since the fibers is decomposed because that the polysaccharides of the sample paper are hydrolyzed by the enzyme action, the bio-kaisen pulp yield does not goes up to over 40%. On the other hand, with the composition according to the fifth aspect of the present invention, a high bio-kaisen pulp yield is obtained by making good use of the available validity of the bio-kaisen composition which disintegrates substantially the PPC paper without hydrolysis of polysaccharides. Further, as for the PPC paper on which the stock market column of a newspaper is copied with a plain paper copier, the most of the ink can not press through the 24 mesh screen, because that the ink on the paper copied is only slightly crumbled at this test conditions. Thus, the bio-kaisen composition of the present invention is also effective for the removal of contaminants such as ink and other adhesive materials.

The bio-kaisen compositions according to the present invention may be prerared by culturing a microbes producing the composition and recovering the composition from the culture. The microbes having an ability to produce the bio-kaisen composition include those that produce cellulolytic enzyme, contained bacteria such as those belong to the genera Bacillus, Cellvibrio, Cellulomonas, Pseudomonas, Clostridium and Ruminococcus; actinomycetes such as those belong to the genera Streptomyces and Thermoactinomyces; fungi such as those belong to the genera Acremonium, Aspergillas, Chaetomium, Coriolus, Fusarium, Humicola, Irpex, Myrothecium, Neurospora, Pellicularia, Penicillium, Phanerochaete, Pleurotus, Polyporus, Poria, Pyricularia, Rhizopus, Schizophyllum, Sclerotium, Scytalidium, Sporotrichum, Talaromyces, Thermoascus, Trametes, and Trichoderma.

Among the above microbes, Trichoderma reesei QM-9414, ATCC 26921, Trichoderma viride IFO 31137, Trichoderma harzianum IFO 31292, and Coriolus versicolor, IFO 4937 were herein employed to obtain the bio-kaisen compositions in the actual examples.

Furthermore, in order to discover the microbes which have high bio-kaisen activity, the present inventors collected various microbes from soil, rotten wood and the like, and screened for the cellulase and bio-kaisen activity. As a result, the present inventors isolated Trichoderma sp. SK-1919 which exhibits high bio-kaisen activity and low cellulase activity. Trichoderma sp. SK-1919 isolated from rotten wood was looked in the log yard of pulp mill in Tomakomai city, Hokkai-do, Japan. Trichoderma sp. SK-1919 has the following mycological properties:

(I) Growth Condition on Various Media

(i) Growth Condition on Malt Extract-Dextrose Agar Medium

This fungi on the malt extract-dextrose agar medium grow rapidly within two days at 30°C, raised colony of 66 to 68 mm. They form at first smooth-surfaced, watery white and sparse mycelial mat, which later become floccose and hairy from the formation of loose scanty aerial hyphae which make the colonies appear somewhat whitish. At maturity the conidial areas are green while the reverse remains uncoloured. The mycelium is composed of hyaline, smooth walled, septate, much branched, 2.5 - 8.0 $\mu$m diameter hyphae.

(ii) Growth Condition on Potato-Dextrose Agar Medium

The fungi on the potato-dextrose agar medium grow rapidly within two days at 30°C, raised colony of 74 to 75 mm. They form at first smooth-surfaced, watery white and sparse mycelial mat, which later become floccose or compactly tufted from the formation of loose aerial hyphae which make the colonies appear whitish. At maturity the conidial areas are green or dark green while the colour of reverse change pale-yellow. The mycelium is composed of hyaline, smooth walled, septate, much branched, 2.5 - 8.0 $\mu$m diameter hyphae. All the branches stand at wide angles to their bearer and their apices are terminated by phialides. Phialides stand at a wide angle to their bearer and are straight or slightly curved, nine-pin-shaped. Phialospores are green-coloured and globose or subglobose, and accumulate at the tip of each phialide to form a globose conidial head.

(iii) Growth Condition on Czapek's Agar Medium

The fungi on the Czapek's agar medium no grows. However, if the pH of this medium is adjusted to 5.0, it slightly grows.

6

(II) Physiological Properties

(i) Temperature range for growth (potato-dextrose agar medium)

The temperature range for the growth is 15° - 40°C and an optimum temperature range is 25° - 35°C. The mycerial growth is maximum at 35°C.

(ii) pH range for growth (potato-dextrose agar medium)

The pH range for the growth is 2.5 - 7.5 and an optimum pH range is 4.5 - 7.0.

From the mycological properties described above, this strain was identified as a strain belonging to genus Trichoderma. Trichoderma sp. SK-1919 was deposited with Fermentation Research Institute of Japan in accordance with the Budapest Treaty under an accession number of FERM BP-3391.

The culture of microbes to obtain the bio-kaisen composition of the present invention may be carried out by a conventional method as well. It is preferred that the medium contain the materials such as lignocellulose or the substances industrially or agriculturally obtained from lignocellulose in which contained cellulose or hemicellulose, and cellulose or hemicellulose of 0.001 - 5% by weight per volume. Using the pulp or wastepaper and corn steep liquor is especially effective for promoting the production of the bio-kaisen composition. The production of the bio-kaisen composition may be stimulated when the subculture is inoculated in an inoculum size of not less than 10%.

The performance of disintegrating and beating newspaper and PPC paper is effectively enhanced by the action of bio-kaisen composition, and these effects are applied to a processing of the various paper such as printing, writing, wrapping and sanitary paper, or of the various paperboad such as container and box boad, or of the lignocellulose such as wood and nonwood including straw, sugar cane bagasse and the like, or of the various pulp such as mechanical, chemical or semichemical pulp. Furthermore, using the bio-kaisen composition may be available to remove ink, film and stickies from the fibers.

Furthermore, the bio-kaisen composition can be used to enhance the bleachability of various pulp such as chemical, semichemical or mechanical pulp. For example, the bio-kaisen composition can be used to decrease the dosage of bleaching chemicals such as chlorine, chlorine dioxide, caustic, hypochlorite, oxygen, ozone, hydrogen peroxide, hydrosulfite, bisulfite and the like, or to elevate the brightness ceiling.

Unlike the conventional cellulolytic enzymes, as the pulp and paper are disintegrated and beated under a condition in which these are no degraded by the bio-kaisen composition, the fibers formed are high quality and high yield. Therefore, the fibers obtained by processing with using the bio-kaisen composition may effectively be utilized in various pulping process of the wastepaper, such as disintegration, beating, deinking, bleach, refinary and the like. Of course, the bio-kaisen composition may effectively be utilized in various pulping process of the mechanical, chemical or semichemical pulp, such as disintegration, beating, bleach, refinary and the like. The composition of the present invention may further be available for the various industrial fields such as, for example, the improvement of cotton cellulose, the saccharification of lignocellulose or the treatment of urban and agricultural waste.

Although the bio-kaisen composition can disintegrate or beat lignocellulose by oneself, needless to say, the composition may be employed together with the conventional pulping process by physical and chemical action.

The invention will now be described by way of example thereof. It should be noted that the examples are presented for the illustration purpose only and should not be interpreted in any restrictive way.

Comparative Example 1

The bio-kaisen activities in terms of newspaper bio-kaisen rate of various conventional cellulolytic enzymes were measured according to the method described above after these enzymes appropriately dilute. CM-cellulase activity was measured by a conventional method described in "Methods in Enzymology" (1988), Vol.160, pp.275 - 299, published by Academic Press, New York. 0.5. ml of the substrate solution prepared by soluting 1 g of CM-cellulose (from wako pure chemical Industries, Osaka) in 100 ml of the acetate buffer, pH 5.0, was put in a test tube and preincubated at 37°C for 10min. Then, 0.5 ml of the enzyme solution appropriately with the acetate buffer was added to initiate the reaction. After 10 min at 37°C, the reaction was terminated by adding 1.0 ml of copper reagent. The tubes, together with a blank in which copper reagent was added before the enzyme, were closed with glass beads, and heated on a vigorously boiling water bath for 15 min. The samples were cooled with tap water, then 1.0 ml of arsenomolybdate reagent was added, and the mixture left for 15 min. The samples were diluted to 10 ml with water, and the absorbance of samples at 500 nm was measured. Xylanase activity was assayed as for CM-cellulase activity except for using 1% of birchwood xylan (from sigma) as a substrate. One unit (IU) of each enzyme activity was defined as the amount of enzyme that releases 1 $\mu$mol of reducing sugar as glucose per minute under the assay condition. The results assayed were expressed in terms of the cellulolytic activities needed for the activity of the newspaper bio-kaisen rate of 50%, which are shown in Table 1.

Table 1

| Cellulolytic Activity Needed for Attaining the Activity of 50% of the Newspaper Bio-kaisen Rate on the Conventional Enzymes | | |
|---|---|---|
| Conventional Cellulases | Enzyme Activity (IU) | |
| | CM-cellulase | Xylanase |
| Meicelase | 0.96 | 0.13 |
| Celluclast | 0.58 | 0.04 |
| Celluzyme SP-342 | 0.63 | 1.0 |
| Pulpzyme | 0.40 | 0.76 |
| Cellulase Onozuka | 0.59 | 1.6 |
| Cellulase Y-NC | 6.6 | 1.6 |
| Meicelase (from Meiji Seika Kaisaha, Ltd) | | |
| Celluclast, Celluzyme SP-342 and Pulpzyme (from Novo Nordisk Bioindustry Ltd.) | | |
| Cellulase Onozuka and Cellulase Y-NC (from Yakult Honsha Co., Ltd.) | | |

Comparative Example 2

The bio-kaisen activities in terms of PPC paper bio-kaisen rate of the above enzymes were measured according to the method described above after these enzymes appropriately dilute. The results assayed were expressed in terms of the cellulolytic activities needed for the activity of the PPC paper bio-kaisen rate of 50%, which are shown in Table 2.

Table 2

| Cellulolytic Activity Needed for Attaining the Activity of 50% of the PPC Paper bio-kaisen Rate on the Conventional Enzymes | | |
|---|---|---|
| Conventional Cellulases | Enzyme Activity (IU) | |
| | CM-cellulase | Xylanase |
| Meicelase | 0.23 | 0.03 |
| Celluclast | 0.15 | 0.02 |
| Celluzyme SP-342 | 0.09 | 0.15 |
| Pulpzyme | 0.19 | 0.36 |
| Cellulase Onozuka | 0.11 | 0.3 |
| Cellulase Y-NC | 0.71 | 0.18 |

Comparative Example 3

The bio-kaisen activity in terms of PPC paper bio-kaisen pulp yield of the conventional enzymes was measured by the method described above with using a Bauer-McNett classifier (from Tozai seiki Co., Ltd.). The results assayed were expressed in terms of the cellulolytic activities needed for the activity of each PPC paper bio-kaisen pulp yield, which are shown in Table 3.

Table 3

| Cellulolytic Activity Needed for Attaining the Activity of each PPC Paper Bio-kaisen Pulp yield on the Conventional Enzymes | | | |
|---|---|---|---|
| Conventional Cellulases | Enzyme Activity | | |
| | CM-cellulase (IU) | Xylanase (IU) | Bio-kaisen Pulp Yield (%) |
| Pulpzyme | 290 | 550 | 30 |
| Pulpzyme | 580 | 1100 | 35 |
| Cellulase Onozuka | 300 | 830 | 30 |
| Cellulase Onozuka | 1480 | 4080 | 40 |

Comparative Example 4

The LUKP (unbleached kraft pulp from mixed hadwood, kappa number 17.5 measured by a conventional method described in TAPPI Standard T-236 hm-85) pretreated with Pulpzyme as the enzyme which enhanced the bleachability of pulp was bleached in a five-stage bleaching sequence, IC-2E-3H-4E-5D.

At first, fifty grams of dry pulp diluted to 1800 ml with water were disintegrated for one minute in the standard disintegrator in accordance with TAPPI Standard T-205 om-88, and after the Pulpzyme having 140 IU of CM-cellulase activity and 270 IU of xylanase activity plus the 1M acetate buffer, pH 5.7, of 50 ml were added, dilute the specimens (2.5% pulp consistency) to 2000 ml with water were again disintegrated for one minute. After the specimens were incubated at 45°C for 2 hours, the pulp was washed thoroughly with water on a wire screen. The kappa number of the pulp pretreated with the enzyme became 14.5. As a control, the same LUKP was pretreated identical to the condition described above except for the incubation with only the acetate buffer. The kappa number of the pulp pretreated with only the buffer became 16.3. The yield of pulp pretreated with the enzyme and only the buffer with respect to the original pulp was 90.7% and 98.7%, respectively.

At second, the pretreated pulps were bleached in the Bleaching Condition 1 shown in Table 4.

Table 4

Bleaching Condition

| Sequence | 1C | 2E | 3H | 4E | 5D |
|---|---|---|---|---|---|
| Chemical applied, % | | | | | |
| Cl₂ | below | | | | |
| NaOH | | below | | 0.5 | |
| NaOCl | | | 1.0 | | |
| ClO₂ | | | | | 0.3 |
| Temperature, °C | RT | 60 | 38 | 80 | 70 |
| Time, minute | 60 | 60 | 180 | 60 | 240 |
| Consistency, % | 3 | 10 | 10 | 10 | 10 |

The dosage of chlorine in the 1C stage and NaOH in the 2E stage

Bleaching condition 1: (kappa number × 0.10)% of Cl₂
(kappa number × 0.09)% of NaOH

Bleaching condition 2: (kappa number × 0.15)% of Cl₂
(kappa number × 0.07)% of NaOH

RT is room temperature.

The brightness of the pulp that was bleached in the Bleaching Condition 1 was 87.2% of the pulp pretreated with the Pulpzyme, 85.4% of the pulp pretreated with only the buffer and 85.1% of the pulp unpretreated.

Comparative Example 5

The LUKP (Kappa number 19.4) pretreated with Pulpzyme was bleached in a five-stage bleaching sequence.

At first, one hundred grams of dry pulp diluted to 2000 ml with water were disintegrated for one minute in the standard disintegrator, the pulp was washed with water on a filter paper. After the Pulpzyme having 28 IU of CM-cellulase activity and 54 IU of xylanase activity plus the 1M acetate buffer, pH 5.7, of 50 ml were added, dilute the specimens (5% pulp consistency) to 2000 ml with water were incubated at 45°C for 3 hours. After incubation, the pulp was diluted to about 10 liter with water, and washed thoroughly with water on a filter paper. The kappa number of the pulp pretreated with the enzyme became 18.2. As a control, the same LUKP was pretreated identical to the condition described above except for the incubation with only the acetate buffer. The kappa number of the pulp pretreated with only the buffer became 18.5. The yield of pulp pretreated with the enzyme and only the buffer with respect to the original pulp was 97.9% and 99.7%, respectively.

10

At second, the pretreated pulps were bleached in the Bleaching Condition 2 shown in Table 4.

The brightness of the pulp that was bleached in the Bleaching Condition 2 was 84.6% of the pulp pretreated with the Pulpzyme, 83.0% of the pulp pretreated with only the buffer and 82.8% of the pulp unpretreated. The brightness of the pulp bleached in the Bleaching Condition 1 was 83.8% of the LUKP (kappa number 19.4).

Example 1

The medium, pH 4.5, consisted of the following additions to water (in grams per liter): DIP (newspaper deinked pulp), 5; cellobiose, 3; corn steep liquor, 3; $KH_2PO_4$, 3; $MgSO_4 \cdot 7H_2O$, 0.2; $CaCl_2 \cdot 2H_2O$, 0.5; NaCl, 0.5; $FeSO_4 \cdot 7H_2O$, 0.01. The 40 ml of medium was added to each 100 ml Erlenmeyer flask equipped with the buffle made three dents in the side of flask. The media were autoclaved at 120°C for 20 minuts. The fungi shown in Table 5 were separately inoculated into each medium, and were incubated at 35°C for 4 days on a rotary shaker (5.0 cm excursion diameter, 160 rev/min). After incubation, the each culture medium is centrifuged and made 0.2 mg/ml with respect to $NaN_3$ to prevent bacterial growth. The enzyme activities of the each solution contained the bio-kaisen composition were measured as described in Comparative Example 1. The results assayed the cellulolytic activities and the newspaper bio-kaisen rate are shown in Table 5.

Table 5

| Cellulolytic Activity Needed for Attaining the Activity of 50% of the Newspaper Bio-kaisen Rate on the Culture Medium | | |
|---|---|---|
| Culture medium | Enzyme Activity (IU) | |
| | CM-cellulase | Xylanase |
| T. reesei QM-9414 | 0.04 | 0.42 |
| C. versicolor IFO 4937 | 0.27 | 0.30 |
| T. sp. SK-1919 | 0.33 | 0.45 |

Example 2

The PPC paper bio-kaisen rate of the culture medium as for Example 1 were assayed as described in Comparative Example 2. The results are shown in Table 6.

Table 6

| Cellulolytic Activity Needed for Attaining the Activity of 50% of the PPC Paper Bio-kaisen Rate on the Culture Medium | | |
|---|---|---|
| Culture medium | Enzyme Activity (IU) | |
| | CM-cellulase | Xylanase |
| T. reesei QM-9414 | 0.01 | 0.10 |
| C. versicolor IFO 4937 | 0.09 | 0.10 |
| T. sp. SK-1919 | 0.04 | 0.05 |

Example 3

The medium, pH 5.5, consisted of the following additions to water (in grams per liter): DIP, 2; glucose, 2; corn steep liquor, 1; $(NH_4)_2SO_4$, 0.4; $KH_2PO_4$, 3; $MgSO_4 \cdot 7H_2O$, 0.2; $CaCl_2 \cdot 2H_2O$, 0.5; NaCl, 0.5; $FeSO_4 \cdot 7H_2O$, 0.01. T. sp. SK-1919 was inoculated into the culture medium prepared as for Example 1 except for using the medium of constituent described above, and was incubated at 35°C for 24 hours on a rotary shaker (5.0 cm excursion diameter, 200 rev/min). After incubation, the culture medium was prepared as for Example 1. The enzyme activities of the solution contained the bio-kaisen composition were measured as described in Comparative Example 1. The cellulolytic activities needed for attaining the activity of the newspaper bio-kaisen rate of 50% of the culture medium were CM-cellulase activity of 0.15 IU and xylanase activity of 0.23 IU.

Example 4

The medium, pH 4.5, consisted of the following additions to water (in grams per liter): DIP, 2; lactose, 2; corn steep liquor, 3; $KH_2PO_4$, 3; $MgSO_4 \cdot 7H_2O$, 0.2; $CaCl_2 \cdot 2H_2O$, 0.5; NaCl, 0.5; $FeSO_4 \cdot 7H_2O$, 0.01. T. sp. SK-1919 was inoculated into the culture medium prepared as for Example 1 except for using the medium of constituent described above, and was incubated at 35°C for 3 days on a rotary shaker (5.0 cm excursion diameter, 80 rev/min). After incubation, the culture medium is prepared as for Example 1. The enzyme activity of the solution contained the bio-kaisen composition was measured as described in Comparative Example 2. The cellulolytic activities needed for attaining the activity of the PPC paper bio-kaisen rate of 50% of the culture medium were CM-cellulase activity of 0.03 IU and xylanase activity of 0.29 IU.

Example 5

The subculture medium, pH 5.9, consisted of the following additions to water (in grams per liter): LUKP (bleached kraft pulp from hardwood), 7; corn steep liquor, 3.5; $(NH_4)_2SO_4$, 1.4; $KH_2PO_4$, 2; $MgSO_4 \cdot 7H_2O$, 0.3; $CaCl_2 \cdot 2H_2O$, 0.3; $FeSO_4 \cdot 7H_2O$, 0.1. The medium prepared as for Example 1 except for using the 100 ml of medium of constituent described above and 500 ml Erlenmeyer flask equipped with the buffle made two dents in the side of flask. T. sp. SK-1919 was inoculated into the starter culture medium prerared as for the above method except for using glucose in place of LUKP, and was incubated at 35°C for 36 hours on a rotary shaker (5.0 cm excursion diameter, 200 rev/min). Ten ml of the culture broth obtained was inoculated into the subculture medium, and was incubated for 24 hours in the same condition. After incubation, the culture medium was prerared as for Example 1. The cellulolytic activities needed for attaining the activity of the PPC paper bio-kaisen rate of 50% on the culture medium were CM-cellulase activity of 0.03 IU and xylanase activity of 0.12 IU.

Example 6

The starter culture medium, pH 5.0, consisted of the following additions to water (in grams per liter): glucose, 10; corn steep liquor, 3; $(NH_4)_2SO_4$, 1.5; $KH_2PO_4$, 3; $MgSO_4 \cdot 7H_2O$, 0.2; $CaCl_2 \cdot 2H_2O$, 0.3; NaCl, 0.3; $FeSO_4 \cdot 7H_2O$, 0.01. The subculture medium, pH 6.5, consisted of the following additions to water (in grams per 750 ml): DIP, 2; corn steep liquor, 3; $(NH_4)_2SO_4$, 1.5. The each medium prerared as for Example 5 except for using the each media of constituent described above. The fungi shown in Table 7 were separately inoculated into each starter culture media and incubated at 30°C for 19 hours on a rotary shaker (5.0 cm excursion diameter, 200 rev/min). Twenty five ml of the culture broth obtained was inoculated into the 75 ml or subculture media and was incubated for 2 days in the same condition. After incubation, the each culture medium was prerared as for Example 1. The enzyme activities of the each solution contained the bio-kaisen composition were measured as described in Comparative Example 1. The results assayed the celluloytic activities and the newspaper bio-kaisen rate are shown in Table 7.

Table 7

| Cellulolytic Activity Needed for Attaining the Activity of 50% of the Newspaper Bio-kaisen Rate on the Culture Medium | | |
|---|---|---|
| Culture medium | Enzyme Activity (IU) | |
| | CM-cellulase | Xylanase |
| T. viride IFO 31137 | 0.06 | 0.21 |
| T. harzianum IFO 4937 | 0.07 | 0.37 |
| T. sp. SK-1919 | 0.14 | 0.18 |

The PPC paper bio-kaisen rate of the culture medium were assayed as for Example 2. The results are shown in Table 8.

Table 8

| Cellulolytic Activity Needed for Attaining the Activity of 50% of the PPC Paper Bio-kaisen Rate on the Culture Medium | | |
|---|---|---|
| Culture medium | Enzyme Activity (IU) | |
| | CM-cellulase | Xylanase |
| T. viride IFO 31137 | 0.014 | 0.049 |
| T. harzianum IFO 4937 | 0.011 | 0.058 |
| T. sp. SK-1919 | 0.030 | 0.038 |

Example 7

T sp. SK-1919 was cultured in a 10 liter jar fermentor. The starter culture medium, pH 5.0, consisted of the following additions to water (in grams per liter): glucose, 10; corn steep liquor, 15; $KH_2PO_4$, 5; $MgSO_4 \cdot 7H_2O$, 0.2; $CaCl_2 \cdot 2H_2O$, 0.5; NaCl, 0.5; $FeSO_4 \cdot 7H_2O$, 0.01. This medium prerared as for Example 5 except for using the medium of constituent described above. The fungus was inoculated into the starter culture medium and incubated at 35°C for 15 hours on a rotary shaker (5.0 cm excursion diameter, 200 rev/min). The medium of jar fermentor consisted of the following additions to water (in grams per 6 liter): DIP, 14; corn steep liquor, 70; $(NH_4)_2SO_4$, 28; $KH_2PO_4$, 28; $MgSO_4 \cdot 7H_2O$, 1.4; $CaCl_2 \cdot 2H_2O$, 1.4; NaCl, 1.4; $FeSO_4 \cdot 7H_2O$, 0.07. The media were autoclaved at 120°C for 20 minuts. One liter of the culture broth obtained above was inoculated into a jar fermentor, and the culture was carried out with an air flow of 7 liters per minute and an agitator speed of 200 revolutions per minute at 35°C. The pH of medium was controlled so as not to decrease below 3.0 by addition of 2M $NH_4OH$. After the culture medium became to pH 5.5, it was harvested at 47 hours of incubation and prerared as for Example 1.

The enzyme activities of the solution contained the bio-kaisen composition were measured as described in Comparative Example 3. As shown in Fig. 3 and 4, the bio-kaisen pulp yield reached more than 50%. The CM-cellulase and xylanase activities needed for attaining a bio-kaisen pulp yield of 41% were only 6 IU and 33 IU, respectively, and those needed for attaining the maximum bio-kaisen pulp yield of 52% were only 22 IU and 119 IU, respectively. In Fig. 3, the symbols of "○", "△" and "□" denote the results of the culture medium of Example 7, Pulpzyme and Cellulase Onozuka, respectively. In Fig. 4, the symbols of "●", "▲" and "■" denote the results of the culture medium of Example 7, Pulpzyme and Cellulase Onozuka, respectively.

Example 8

The LUKP (kappa number 17.5) was pretreated in the same manner as in Comparative Example 4 except for using the culture medium obtained Example 7 as the enzyme which enhanced the bleachability of pulp. The kappa number of the pulp pretreated with the culture medium including the bio-kaisen composition having 4.8 IU of CM-cellulase activity and 26 IU of xylanase activity became 14.8. The yield of pulp pretreated with the culture medium with respect to the original pulp was 95.1%.

The pulp was bleached in the same manner as in Comparative Example 4. The brightness of the pulp that was bleached in the Bleaching Condition 1 was 87.8% of the pulp pretreated with the bio-kaisen composition.

Example 9

To perform the starter culture, T. sp. SK-1919 was incubated for 17 hours in the same manner as in Example 7. The medium of jar fermentor consisted of the following additions to water (in grams per 6 liter): DIP. 14; corn steep liquor, 35; glucose, 70; whey powder, 35; $(NH_4)_2SO_4$, 35; $KH_2PO_4$, 21; $MgSO_4 \cdot 7H_2O$, 1.4; $CaCl_2 \cdot 2H_2O$, 2.1; NaCl, 2.1; $FeSO_4 \cdot 7H_2O$, 0.07. The medium was autoclaved at 120°C for 20 minuts. The fungus was cutured in the same manner as in Example 7 except for the incubation with an air flow of one liters per minute and an agitator speed of 300 revolutions per minute at 30°C. After the culture medium became to pH 5.0, it was harvested at 97 hours of incubation and prerared as for Example 1.

The enzyme activities of the solution contained the bio-kaisen composition were measured as described in Comparative Example 1, 2 and 3. The cellulolytic activities needed for attaining the activity of the newspaper bio-kaisen rate of 50% of the culture medium were CM-cellulase activity of 0.10 IU and xylanase activity of 0.36 IU. The cellulolytic activities needed for attaining the activity of the PPC paper bio-kaisen rate of 50% of the culture medium were CM-cellulase activity of 0.015 IU and xylanase activity of 0.053 IU. The CM-cellulase and xylanase activities needed for attaining a bio-kaisen pulp yield of 41% were 33 IU and 118 IU, respectively, and those needed for attaining the maximum bio-kaisen pulp yield of 48% were 66 IU and 235 IU, respectively.

The LUKP (kappa number 17.5) was pretreated in the same manner as in Example 9 except for using the culture medium obtained above as the enzyme which enhanced the bleachability of pulp. The kappa number of the pulp pretreated with the culture medium including the bio-kaisen composition having 3.4 IU of CM-cellulase activity and 12 IU of xylanase activity became 15.2. The yield of pulp pretreated with the culture medium with respect to the original pulp was 95.2%.

The pulp was bleached in the same manner as in Comparative Example 4. The brightness of the pulp that was bleached in the Bleaching Condition 1 was 87.6% of the pulp pretreated with the bio-kaisen composition.

Example 10

The starter culture medium, pH 6.0, consisted of the following additions to water (in grams per liter): glucose, 10; whey powder, 40; corn steep liquor, 50; $(NH_4)_2SO_4$, 8; $KH_2PO_4$, 5; $MgSO_4 \cdot 7H_2O$, 0.1; NaCl, 0.1. The subculture medium, pH 5.8, consisted of the following additions to water (in grams per 750 ml): DIP, 10; whey powder, 20; corn steep liquor, 10; $(NH_4)_2SO_4$, 5. The each medium prerared as for Example 5 except for using the each medium of constituent described above and the 150 ml of the subculture. T. sp. SK-1919 was inoculated into the starter culture medium and incubated at 30°C for 17 hours on a rotary shaker (5.0 cm excursion diameter, 200 rev/min). Fifty ml of the culture broth obtained was inoculated into the 150 ml of subculture medium and was incubated for 78 hours in the same culture condition. After incubation, the culture medium was prerared as for Example 1.

The enzyme activities of the solution contained the bio-kaisen composition were measured as described in Example 9. The cellulolytic activities needed for attaining the activity of the newspaper bio-kaisen rate of 50% of the culture medium were CM-cellulase activity of 0.23 IU and xylanase activity of 1.8 IU. The cellulolytic activities needed for attaining the activity of the PPC paper bio-kaisen rate of 50% of the culture medium were CM-cellulase activity of 0.031 IU and xylanase activity of 0.25 IU.

The LUKP (kappa number 19.4) was pretreated in the same manner as in Comparative Example 5 except for using the culture medium obtained above as the enzyme which enhanced the bleachability of pulp. The kappa number of the pulp pretreated with the culture medium including the bio-kaisen composition having 4.2 IU of CM-cellulase activity and 33 IU of xylanase activity became 17.7. The yield of pulp pretreated with the culture medium with respect to the original pulp was 98.5%.

The pulp was bleached in the same manner as in Comparative Example 5. The brightness of the pulp that was bleached in the Bleaching Condition 2 was 85.4% of the pulp pretreated with the bio-kaisen composition.

Example 11

T. reesei QM-9414 was inoculated into the starter culture medium and incubated as for the culture condition of Example 9. The subculture medium, pH 5.8, consisted of the following additions to water (in grams per 750 ml): DIP, 10; corn steep liquor, 5; $(NH_4)_2SO_4$, 2. The medium prerared as for Example 10 except for using the medium of constituent described above. The fungus was incubated for 78 hours in the same culture condition of Example 9. After incubation, the culture medium was prerared as for Example 1.

The enzyme activities of the solution contained the bio-kaisen composition were measured as described in Example 10. The cellulolytic activities needed for attaining the activity of the newspaper bio-kaisen rate of 50% of the culture medium were CM-cellulase activity of 0.17 IU and xylanase activity of 1.2 IU. The cellulolytic activities needed for attaining the activity of the PPC paper bio-kaisen rate of 50% of the culture medium were CM-cellulase activity of 0.043 IU and xylanase activity of 0.31 IU.

The LUKP (kappa number 19.4) was pretreated in the same manner as in Example 10 except for using the culture medium obtained above as the enzyme which enhanced the bleachability of pulp. The kappa number of the pulp pretreated with the culture medium including the bio-kaisen composition having 7.9 IU of CM-cellulase activity and 56 IU of xylanase activity became 17.6. The yield of pulp pretreated with the culture medium with respect to the original pulp was 97.8%.

The pulp was bleached in the same manner as in Example 10. The brightness of the pulp that was bleached in the Bleaching Condition 2 was 86.0% of the pulp pretreated with the bio-kaisen composition.

**Claims**

1.  A bio-kaisen composition having each CM-cellulase and xylanase activity of not more than 0.5 IU and a bio-kaisen activity in terms of the newspaper bio-kaisen rate of not less than 50%.

2.  A bio-kaisen composition having a CM-cellulase activity of not more than 0.35 IU and a bio-kaisen activity in terms of the newspaper bio-kaisen rate of not less than 50%.

3.  A bio-kaisen composition having each CM-cellulase and xylanase activity of not more than 0.14 IU and a bio-kaisen activity in terms of the PPC paper bio-kaisen rate of not less than 50%.

4.  A bio-kaisen composition having a CM-cellulase activity of not more than 0.05 IU and a bio-kaisen activity in terms of the PPC paper bio-kaisen rate of not less than 50%.

5.  A bio-kaisen composition having a bio-kaisen activity in terms of the bio-kaisen pulp yield determined by the PPC paper bio-kaisen pulp recovery test of not less than 43%.

6.  A bio-kaisen composition having each CM-cellulase and xylanase activity of not more than 400 IU and a bio-kaisen activity in terms of said the bio-kaisen pulp yield of not less than 30%.

7.  A bio-kaisen composition having a CM-cellulase activity of not more than 200 IU and a bio-kaisen activity in terms of said the bio-kaisen pulp yield of not less than 30%.

8.  A process of producing said bio-kaisen composition of any one of claims 1 - 7, comprising the steps of culturing a microbe belonging to genera Trichoderma and Coriolus, which have an ability to produce said bio-kaisen composition of any one of claims 1 - 7, and of recovering said bio-kaisen composition produced.

9.  The process of claim 8, wherein the culturing of the microbe is carried out in the presence of material contained cellulose or hemicellulose.

10. The process of claim 8, wherein the culturing of the microbe is carried out in the presence of wastepaper, secondary fiber prepared from wastepaper or pulp from lignocellulose.

**11.** A method of processing said paper or pulp comprising the step of treating said paper or pulp with said bio-kaisen composition according to any one of claims 1 - 7.

**12.** A method of recovering of the fibers obtained by processing said paper or pulp comprising the step of treating said paper or pulp with said bio-kaisen composition according to any one of claims 1 - 7.

**13.** The method of claim 11, wherein processing said paper or pulp comprising the step of deinking said paper or pulp with said bio-kaisen composition.

**14.** The method of claim 12, wherein said recovering of the fibers obtained by processing said paper or pulp comprising the step of deinking said paper or pulp with said bio-kaisen composition.

**15.** The method of claim 11, wherein processing said paper or pulp comprising the step of bleaching said paper or pulp with said bio-kaisen composition.

**16.** The method of claim 12, wherein said recovering of the fibers obtained by processing said paper or pulp comprising the step of bleaching said paper or pulp with said bio-kaisen composition.

Fig. 1

Fig. 2

Fig. 3

Fig. 4

**European Patent Office**

**PARTIAL EUROPEAN SEARCH REPORT**
which under Rule 45 of the European Patent Convention
shall be considered, for the purposes of subsequent
proceedings, as the European search report

Application number

EP 91304636
– page 1 –

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | TAPPI JOURNAL vol. 73, no. 4, April 1990, Norcross, US, pages 198-204; P.C. TROTTER: "Biotechnology in the pulp and paper industry: a review. Part 1: tree improvement, pulping and bleaching, and dissolving pulp applications" * the whole document * | 8-15 | D21H21/32 D21C9/10 D21C9/00 D21C5/02 D21C3/00 |
| X | DERWENT BIOTECHNOLOGY ABSTRACTS DATABASE Derwent Publications Ltd., London, GB; POURQUIE J. et al.: "Scale-up of cellulase production and utilization- Trichoderma reesei culture" & FEMS SYMP.43,71-86,1988 | 8,9 | |
| A | WO-A-8700564 (REPLIGEN CORPORATION) * page 3, lines 3-16; page 7, line 15 - page 8, line 25 * | 8,11-16 | |
| A | EP-A-351655 (CULTOR LTD.) * page 1, line 49 - page 4, line 9 * | 8,11, 12 | **TECHNICAL FIELDS SEARCHED (Int. Cl.5)** |

TECHNICAL FIELDS SEARCHED (Int. Cl.5)

D21H
D21C
C12N

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 8-16
Claims searched incompletely:
Claims not searched: 1-7
Reason for the limitation of the search.

Lack of technical disclosure of the exact structural elements of the composition

| Place of search | Date of completion of the search | Examiner |
|---|---|---|
| BERLIN | 12.09.1991 | GURDJIAN, D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1. 03.82

| Category | Citation of document with indication, where appropriate, of relevant passages | Relevant to claim | CLASSIFICATION OF THE APPLICATION (Int. Cl.) |
|---|---|---|---|
| **DOCUMENTS CONSIDERED TO BE RELEVANT** | | | |
| A | DE-A-3636208 (CALL, HANS-PETER)<br>* abstract * | 1,11-15 | |
| A | GB-A-2231595 (KOREA RESEARCH INSTITUTE OF CHEMICAL TECHNOLOGY)<br>* abstract * | 14 | |
| A | TAPPI JOURNAL<br>vol. 70, no. 10, October 1987, Norcross, US, pages 136-137; M. KOUROS: "Bio-technology in the paper industry-a measured optimism"<br>* the whole document * | 8-15 | |
| A | ABSTRACT BULLETIN OF THE INSTITUTE OF PAPER CHEMISTRY<br>vol. 59, no. 6, December 1988, Appleton, US, page 644; T. NISHIDA et al.:<br>"Lignin biodegradation by wood-rotting fungi.(1). Screening of lignin-degrading fungi"<br>* abstract *<br>& J. Japan Wood Res. Soc. (Mokuzai Gakkaishi) vol. 34, 1988, no. 6, pages 530-536 | 8 | **TECHNICAL FIELDS SEARCHED (Int. Cl.)** |